(19) Europäisches Patentamt
European Patent Office
Office européen des brevets

(11) Publication number : 0 273 731 B1

(12) # EUROPEAN PATENT SPECIFICATION

(45) Date of publication of patent specification : 05.06.91 Bulletin 91/23

(51) Int. Cl.$^5$ : C08G 73/12, C08G 59/40, C07D 487/04, C07D 207/40, C07D 209/56, C08K 5/34, C09J 179/04

(21) Application number : 87311429.2

(22) Date of filing : 24.12.87

(54) **Imide compound and composition containing the same.**

(30) Priority : 26.12.86 JP 314259/86
26.12.86 JP 314260/86
23.06.87 JP 157329/87
23.06.87 JP 157330/87
23.06.87 JP 157331/87

(43) Date of publication of application : 06.07.88 Bulletin 88/27

(45) Publication of the grant of the patent : 05.06.91 Bulletin 91/23

(84) Designated Contracting States : CH DE FR GB IT LI SE

(56) References cited :
CH-A- 445 510
CHEMICAL ABSTRACTS, vol. 67, July 3-24,1967, Columbus, Ohio, USA; T. WAGNER-JAUREGG: "Structure of the addition product of N-butylmaleimide to styrene" pages 1067-1068, abstract-no. 11318u
CHEMICAL ABSTRACTS, vol. 85, July 5-19, 1976, Columbus, Ohio, USA;G. AZIZ et al.: "Diels-Alder reaction: addition of N-naphthylmaleimides to 1,1-diarylethylenes and their 2-halo derivatives", page 638, column 2, abstract-no. 20789x

(73) Proprietor : SUMITOMO CHEMICAL COMPANY, LIMITED
Kitahama 4-chome 5-33
Chuo-ku Osaka 541 (JP)

(72) Inventor : Saito, Yasuhisa
1-2, Gojocho
Higashiosaka, Osaka-fu (JP)
Inventor : Kamio, Kunimasa
24-1-1108, Shinashiya Ue
Suita, Osaka-fu (JP)
Inventor : Shibata, Mitsuhiro
2-105-303, Kyuhoen
Yao, Osaka-fu (JP)
Inventor : Watanabe, Katsuya
1-18 1-112, Tamagawa
Takatsuki, Osaka-fu (JP)
Inventor : Shiomi, Yutaka
8-30, Korigaoka
Hirakata, Osaka-fu (JP)
Inventor : Ueda, Youichi
1968, Misecho
Kashigara, Nara-ken (JP)

(74) Representative : Geering, Keith Edwin et al
REDDIE & GROSE 16 Theobalds Road
London WC1X 8PL (GB)

EP 0 273 731 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to thermosettable imide compounds having terminal functional groups and to resin compositions produced with these imide compounds.

Hitherto, for laminating and encapsulating semiconductor elements such as IC, LSI, etc., epoxy resins have been used.

However, cured epoxy resin products are of low thermal resistance, and this results in epoxy-laminated products suffering large dimensional change perpendicular to the substrate, giving problems such as low through-hole reliability, smear, etc. With epoxy encapsulant for IC, LSI, etc., there was also a problem that when such parts are connected to circuits by soldering, cracks are formed by the resultant heating because of the large thermal expansion of the material. For these reasons, improvement in the thermal resistance of the cured product has been desired.

For improving the thermal resistance of such hardened products, use of aromatic imide compounds as hardener has been proposed.

Generally, aromatic imide compounds are produced from aromatic tetracarboxylic acid anhydrides and aromatic diamines.

The well-known representative aromatic tetracarboxylic acid anhydrides include pyromellitic acid anhydride and benzophenonetetracarboxylic acid anhydride. The aromatic imide compounds obtained with these acid anhydrides, however, have poor compatibility with epoxy resins, so that it was difficult to use them as hardeners for epoxy resins. Also, these aromatic imide compounds have very low solubility in the common low-boiling organic solvents, special high-boiling solvents such as dimethylformamide, dimethylacetamide, N-methylpyrrolidone, dimethyl sulfoxide, cresol, etc. being necessary ; this made it additionally difficult to use the imide compounds together with epoxy resins.

We have found that imide compounds (B) having structural units represented by general formula.

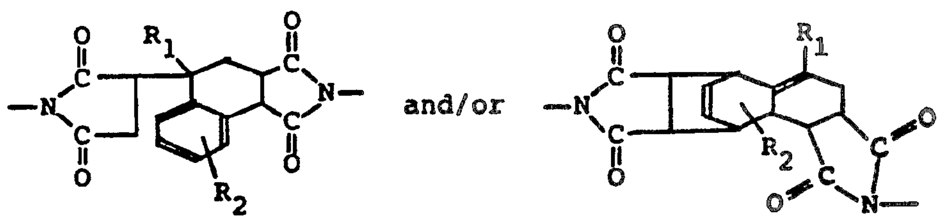

(wherein the $R_1$'s are selected from a hydrogen and $C_1$ to $C_{10}$ alkyl groups and the $R_2$'s are selected from a hydrogen atom and hydroxyl and $C_1$ to $C_{20}$ alkyl and $C_1$-$C_{20}$ alkoxy groups) in the molecule are easily soluble in various organic solvents and of good compatibility with epoxy resins – and also that, by use of such imide compounds in epoxy resins, the foregoing problems such as low thermal resistance, large change of dimension and cracking by the action of heat can be avoided.

We have also found that whereas conventional thermosetting polyimide resins have poor water resistance and adhesion to metallic surfaces, resin compositions comprising the above imide compounds (B) and epoxy resins have adhesion properties and water resistance equivalent to or higher than those of epoxy resins.

Thus, the present invention provides a thermosettable imide compound of formula (I),

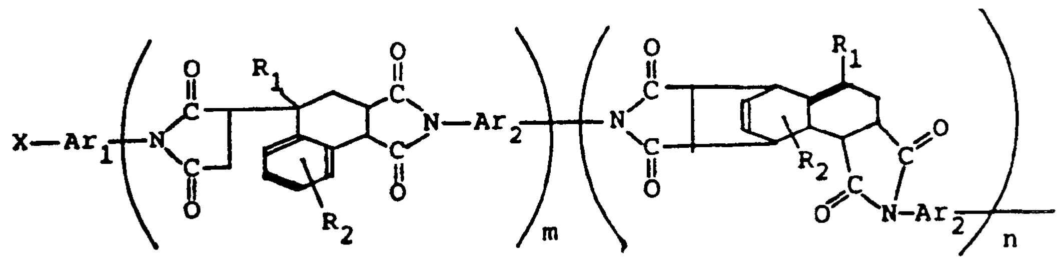

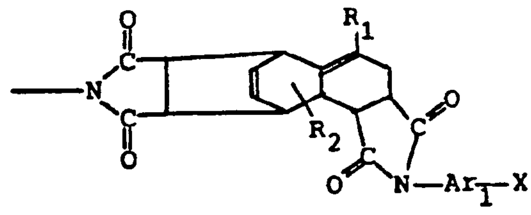

(I)

wherein each X is selected from $-NH_2$ and $-OH$ groups ; each $AR_1$ and each $Ar_2$ is selected independently from aromatic residues ; each $R_1$ is selected from a hydrogen atom and $C_1$-$C_{10}$ alkyl groups, each $R_2$ is selected from a hydrogen atom and hydroxyl and $C_1$-$C_{20}$ alkyl and $C_1$-$C_{20}$ alkoxy groups ; m is 0 or a number up to 30, and n is 0 or a number up to 30.

The present invention also provides epoxy resin compositions (which may for example be adhesive compositions) containing as essential components epoxy resin(s) (A) and imide compound(s) (B) of formula (I) ; and hardened products of these epoxy resin compositions have excellent thermal resistance.

The present invention further provides epoxy resin compositions comprising epoxy resin(s) (A), imide compound(s) (B) of formula (I) and polymaleimide compound(s) (C) having two or more maleimide groups in the molecule.

The present invention also provides epoxy resin compositions containing as essential components epoxy resin(s) (A), imide compound(s) (B) of formula (I) and compound(s) (D) having two or more phenolic -OH groups in the molecule (hereinafter referred to as polyphenol compounds), optionally together with polymaleimide compound(s) (C).

Each $Ar_1$ and each $Ar_2$ in formula (I) is independently a mononuclear or polynuclear divalent aromatic residue of which the aromatic ring may be unsubstituted or substituted – e.g. with a lower alkyl group, a halogen atom, a lower alkoxy group, etc. More specifically, each $Ar_1$ and $Ar_2$ is an aromatic amine residue, any $Ar_2$ being a diamine residue and any $Ar_1$ being a monoamine or diamine residue. Of these aromatic amines, the aromatic diamines include 4,4′-diaminodiphenylmethane, 3,3′-diaminodiphenylmethane, 4,4′-diaminodiphenyl ether, 3,4′-diaminodiphenyl ether, 4,4′-diaminodiphenylpropane, 4,4′-diaminodiphenyl sulfone, 3,3′-diaminodiphenyl sulfone, 2,4-tolylenediamine, 2,6-tolylenediamine, m-phenylenediamine, p-phenylenediamine, benzidine, 4,4′-diaminodiphenyl sulfide, 3,3′-dichloro-4,4′-diaminodiphenyl sulfone, 3,3′-dichloro-4,4′-diaminodiphenylpropane, 3,3′-dimethyl-4,4′-diaminodiphenylmethane, 3,3′-dimethoxy-4,4′-diaminobiphenyl, 3,3′-dimethyl-4,4′-diaminobiphenyl, 1,3-bis(4-aminophenoxy)benzene, 1,3-bis(3-aminophenoxy)benzene, 1,4-bis(4-aminophenoxy)benzene, 2,2-bis(4-aminophenoxyphenyl)propane 4,4′-bis(4-aminophenoxy)diphenyl sulfone, 4,4′-bis(3-aminophenoxy)diphenyl sulfone, 9,9′-bis(4-aminophenyl)anthracene, 9,9′-bis(4-aminophenyl)-fluorene, 3,3′-dicarboxy-4,4′-diaminodiphenylmethane, 2,4-diaminoanisole, bis(3-aminophenyl)methylphosphine oxide, 3,3′-diaminobenzophenone, c-toluidine sulfone, 4,4′-methylene-bis-o-chloroaniline, tetrachlorodiaminodiphenylmethane, m-xylylenediamine, p-xylylenediamine, 4,4′-diaminostilbene, 5-amino-1-(4′-aminophenyl)-1,3,3-trimethylindane, 6-amino-1-(4′-aminophenyl)-1,3,3-trimethylindane, 5-amino-6-methyl-1-(3′-amino-4′-methylphenyl)-1,3,3-trimethylindane, 7-amino-6-methyl-1-(3′-amino-4′-methylphenyl)-1,3,3-trimethylindane, 6-amino-5-methyl-1-(4′-amino-3′-methylphenyl)-1,3,3-trimethylindane and 6-amino-7-methyl-1-(4′-amino-3′-methylphenyl)-1,3,3-trimethylindane. These amine residues may be present alone or in combination.

The aromatic monoamines include o-aminophenol, m-aminophenol, p-aminophenol, 6-amino-m-cresol, 4-amino-m-cresol, 2,2-(4-hydroxyphenyl-4-aminophenyl)propane, 2,2-(4-hydroxyphenyl-2′-methyl-4′-aminophenyl)propane, 2,2-(3-methyl-4-hydroxyphenyl-4′-aminophenyl)propane, 3-amino-1-naphthol, 8-amino-2-naphthol, 5-amino-1-naphthol and 4-amino-2-methyl-1-naphthol. These amine residues may be present alone or in combination.

Preferably, each $R_1$ is selected from alkyl groups of from 1 to 3 carbon atoms ; each $R_2$ is preferably selected from a hydrogen atom and alkyl groups of 1 to 5 carbon atoms.

In formula (I), m and n are the same or different and are preferably numbers of from 0 to 8, most preferably from 0 to 5.

Compounds (I) in which X is $-NH_2$ may be synthesized by reacting, according to the common imidation technique, excess of foregoing aromatic diamine(s) with compound(s) B, of formula Y and/or Z below

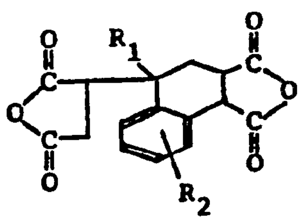

Y

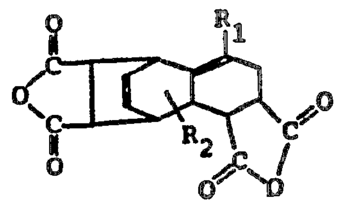

Z

wherein $R_1$ and $R_2$ are as defined above.

Those in which X is -OH may be synthesized by adding foregoing aromatic monoamine(s) having an -OH group and foregoing aromatic diamine(s) to $B_1$ so that the molar ratio of aromatic diamine to $B_1$ is (m + n) : (m + n + 1) and the molar ratio of the aromatic monoamine to $B_1$ is 2 : (m + n + 1) (wherein m and n are as defined above) ; the reaction is according to the common imidation technique.

Methods of preparing the functional group-terminated imide compounds (I) are of course not limited to those illustrated above.

Compounds $B_1$ are obtainable by reacting compounds $B_3$

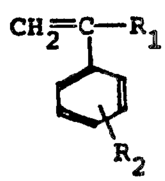

$B_3$

(wherein $R_1$ and $R_2$ are as defined above) with maleic anhydride at a former to latter molar ratio of 1 : 2 in the absence of radical polymerization catalyst and in the presence or absence of radical polymerization inhibitor. Examples of $B_3$ include styrene, α-methylstyrene, α-p-dimethylstyrene, α-m-dimethylstyrene, isopropylstyrene, vinyltoluene, p-tert-butylstyrene, p-isopropenylphenol, m-isopropenylphenol, 1-methoxy-3-isopropenylbenzene, 1-methoxy-4-isopropenylbenzene and vinylxylene. These compounds may be used alone or in combination.

The functional group-terminated imide compounds (I) are soluble in high concentrations in low-boiling solvents such as acetone, methyl ethyl ketone, methyl isobutyl ketone, methyl Cellosolve®, ethyl Cellosolve®, methylene chloride or chloroform, and also they are of superior compatibility with epoxy resins. Consequently, thermo-setting is possible by combining the imide compounds and epoxy resins.

The epoxy resins (A) used in the compositions of the present invention are compounds having two or more epoxy groups in the molecule. Examples of the epoxy resins include glycidyl ether compounds derived from polyhydric phenols [e.g. bisphenol A, bisphenol F, hydroquinone, resorcinol, phloroglucinol, tris(4-hydroxyphenyl)methane, 1,1,2,2-tetrakis(4-hydroxyphenyl)ethane] or halogenated polyphenols (e.g. tetrabromobisphenol A, brominated phenol novolak) ; novolak type epoxy resins derived from novolak resins which are reaction products of phenols (e.g. phenol, o-cresol) with formaldehyde ; amine type epoxy resins derived from aniline, p-aminophenol, m-aminophenol, 4-amino-m-cresol, 6-amino-m-cresol, 4,4'-diaminodiphenylmethane, 3,3'-diaminodiphenylmethane, 4,4'-diaminodiphenyl ether, 3,4'-diaminodiphenyl ether, 1,4-bis(4-aminophenoxy)-benzene, 1,4-bis(3-aminophenoxy)benzene, 1,3-bis(4-aminophenoxy)-benzene, 1,3-bis(3-aminophenoxy)benzene, 2,2-bis(4-aminophenoxyphenyl)-propane, p-phenylenediamine, m-phenylenediamine, 2,4-tolylenediamine, 2,6-tolyenediamine, p-xylylenediamine, m-xylylenediamine, 1,4-cyclohexane-bis(methylamine), 1,3-cyclohexane-bis(methylamine), 5-amino-1-(4'-aminophenyl)-1,3,3-trimethylindane and 6-amino-1-(4'-aminophenyl)-1,3,3-trimethylindane ;

glycidyl ester compounds derived from aromatic carboxylic acids (e.g. p-oxybenzoic acid, m-oxybenzoic acid, terephthalic acid, isophthalic acid) ; hydantoin type epoxy resins derived from 5,5-dimethylhydantoin ; alicyclicepoxy resins such as 2,2'-bis(3,4-epoxycyclohexyl)propane, 2,2-bis[4-(2,3-epoxypropyl)cyclohexyl]propane, vinylcyclohexene dioxide and 3,4-epoxycyclohexylmethyl-3,4-epoxycyclohexanecarboxylate ; and other compounds such as triglycidyl isocyanurate or 2,4,6-triglycidoxy-S-triazine. These epoxy resins may be used alone or in any combination.

As to the proportions of epoxy resin (A) and functional group-terminated imide compound (B), it is preferred that the sum of (B) and any hardener described later is from 0.6 to 1.2 gram equivalent per 1 gram equivalent of (A).

In order to attain further improvement in the thermal resistance of the cured product of the compositions comprising epoxy resin(s) (A) and imide compound(s) (B), compound(s) having two or more maleimide groups (II)

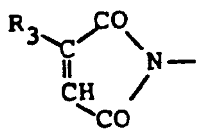

(II)

(wherein $R_3$ represents a hydrogen atom or a lower alkyl group) in the molecule [i.e. polymaleimide compounds(s) (C)] may be incorporated in said composition.

Specific examples of the polymaleimide compounds (C) include N,N'-bismaleimide compounds such as N,N'-diphenylmethane bismaleimide, N,N'-phenylene bismaleimide, N,N'-diphenylether bismaleimide, N,N'-diphenylsulfone bismaleimide, N,N'-dicyclohexylmethane bismaleimide, N,N'-tolylene bismaleimide, N,N'-xylylene bismaleimide, N,N'-diphenylcyclohexane bismaleimide, N,N'-dichlorodiphenylmethane bismaleimide, N,N'-diphenylcyclohexane bismaleimide, N,N'-diphenylmethane bis(methylmaleimide), N,N'-diphenylether bis(methylmaleimide), N,N'-diphenylsulfone bis(methylmaleimide), isomers of these compounds, N,N'-ethylene bismaleimide, N,N'-hexamethylene bismaleimide, N,N'-hexamethylene bis(methylmaleimide) ; prepolymers having a terminal N,N'-bismaleimide skeleton obtained by the reaction of these N,N'-bismaleimide compounds with diamines ; and the maleimidated or methylmaleimidated compounds of aniline formalin polycondensation products.

Particularly, N,N'-diphenylmethane bismaleimide and N,N'-diphenylether bismaleimide are preferred. In the resin compositions of the present invention comprising epoxy resins(s) (A), imide compound(s) (B) and polymaleimide compound(s) (C), it is generally preferred that the proportions of epoxy resin (A) and imide compound (B) give from 0.6 to 1.2 gram equivalent of (B) per 1 gram equivalent of (A), that the proportions of polymaleimide compound (C) and imide compound (B) are such that the number of active hydrogen atoms H in the terminal functionl group X of (B) is 0.6 to 1 per 1 double bond in (C), and that the weight ratio (A)/(C) is from 95/5 to 40/60. However, when other epoxy hardeners described later are used, it is preferred that the sum of compound (B) plus such other hardener is from 0.6 to 1.2 gram equivalent per 1 gram equivalent of (A).

The foregoing composition comprising epoxy resin(s) (A) and imide compound(s) (B), or epoxy resin(s) (A), imide compound(s) (B) and polymaleimide compound(s) (C) may incorporate polyphenol compound(s) (D) having two or more phenolic –OH groups in the molecule ; this may improve the extent and rate of hardening of the compositions.

Examples of such polyphenol compounds include polydihydric phenols [e.g. bisphenol A, bisphenol F, hydroquinone, resorcinol, phloroglucinol, tris(4-hydroxyphenyl)methane, 1,1,2,2-tetrakis(4-hydroxphenyl)ethane] ; halogenated bisphenols (e.g. tetrabromobisphenol A) ; and novolak type condensation products which are reaction products of phenols [e.g. phenol, cresols (including isomers), xylenols (including isomers), hydroquinone, resorcinol, p-tert-butylphenol, p-tert-octylphenol, allylphenols (including isomers), bisphenol A or vinylphenol] and formaldehyde.

As to the proportions of the components of the resin composition of the present invention comprising epoxy resin(s) (A), imide compound(s) (B) and polyphenol compound(s) (D), it is generally preferred that the ratio of the number of active hydrogen atoms in the terminal functional group X of (B) to the number of –OH groups in (D), i.e. (B)/(D), is from 2/1 to 4/1, and that the sum of the active hydrogen equivalents of (B) and (D) is from 0.6 to 1.2 gram equivalent per 1 gram equivalent of (A).

However, when other epoxy hardeners described later are used, it is preferred that the sum of (B), (D) and such other hardener is from 0.6 to 1.2 gram equivalent per 1 gram equivalent of (A).

As to the proportions of the components of the resin composition comprising epoxy resin(s) (A), imide compound(s) (B), polymaleimide compound(s) (C) and polyphenol compound(s) (D), it is generally preferred that

the sum of the active hydrogen equivalent of (B) and (D) is from 0.6 to 1.2 gram equivalent per 1 gram equivalent of (A), and the ratio of the active hydrogen equivalent of (B) to that of (D), i.e. (B)/(D), is from 2/1 to 4/1.

However, when other epoxy hardeners described later are used, it is preferred that the sum of (B), (D) and such other hardener is from 0.6 to 1.2 gram equivalent per 1 gram equivalent of (A).

It is preferred to select the polymaleimide compound(s) (C) so as to be from 2.5 to 30% of the total weight.

Epoxy resin compositions of the present invention can contain epoxy resin(s) (A), functional group-terminated imide compound(s) (B) and optionally polymaleimide compound(s) (C) and/or polyphenol compound(s) (D), all of which are explained above ; they may also contain known epoxy hardeners and cure accelerators, fillers, flame retardants, reinforcing agents, surface-treating agents, pigments, etc. as need arises.

Such known epoxy hardeners include amine type hardeners such as aromatic amines (e.g. xylylenediamine) and aliphatic amines, acid anhydrides, dicyandiamide and hydrazide compounds.

The cure accelerators include amines [e.g. benzyldimethylamine, 2,4,6-tris(dimethylaminomethyl)phenol, 1,8-diazabicycloundecene], imidazole compounds (e.g. 2-ethyl-4-methylimidazole) and boron trifluoride amine complexes. The fillers include silica, calcium carbonate, etc ; the flame retardants include aluminum hydroxide, antimony trioxide and red phosphorus ; and the reinforcing agents include fabrics comprising glass fibers or organic fibers (e.g. polyester fibers, polyamide fibers), alumina fibers, non-woven fabrics, mats, papers and combinations thereof.

Epoxy resin compositions according to the present invention give hardened products having extremely high thermal resistance and are therefore of very high industrial value as material for lamination and molding. They also have excellent adhesion to metallic surfaces, and water resistance.

For example, epoxy resin copper-clad laminates produced with epoxy resin compositions of the present invention have very high thermal resistance, and besides they have excellent adhesion to copper foil and water resistance. Such epoxy resin copper-clad laminates can be obtained by impregnating a substrate with an organic solvent solution of an epoxy resin composition of the present invention, removing the solvent by drying to prepare a prepreg and heat-laminating the prepreg and copper foil. Suitable substrates for laminating include fabrics comprising inorganic fibers (e.g. glass fibers) or organic fibers (e.g. polyester fibers, polyamide fibers), non-woven fabrics, mats, papers and combination thereof. Suitable organic solvents include acetone, methyl ethyl ketone, methyl isobutyl ketone, methyl Cellosolve®, ethyl Cellosolve®, and dimethylformamide. Heat molding is for example by press molding carried out at a temperature of from 150° to 250°C under a pressure of from 10 to 100 kg/cm$^2$ for from 20 to 300 minutes.

The compositions comprising epoxy resin(s) (A), imide compound(s) (B) and polymaleimide compound(s) (C) also have particularly high thermal resistance.

Compositions comprising epoxy resin(s) (A), imide compound(s) (B)and polyphenol compound(s) (D)have high hardening rate, being of very high industrial value.

As described above, compositions of the present invention comprising epoxy resin(s) (A) and imide compound(s) (B) give hardened products having high thermal resistance and adhesion property, and are usable as adhesives and of high industrial value. When a composition is used as an industrial adhesive, it may be kneaded on a roll (e.g. at a temperature of from 110° to 50°C) to prepare a uniform mixture which is then formed into sheet, and the sheet used as the adhesive ; instead the composition may be dissolved in a solvent such as dimethylformamide, methyl Cellosolve® or acetone and the resulting solution directly coated onto adherends – or impregnated into a reinforcing material such as glass fibers and the material dried (at e.g. from 150° to 180°C for from 5 to 10 minutes) to prepare a prepreg which is then used as the adhesive.

The imide compound(s) (B) can also be used in systems containing no epoxy resins. In this case, the fillers, flame retardants, reinforcing agents, surface-treating agents, pigments, etc. described above can also be used as need arises.

Compositions of the present invention comprising imide compound(s) (B) and polymaleimide(s) compound (C) are thermosetting resin compositions which need no high-temperature prolonged heating for of molding ; they are superior in moldability as well as thermal resistance and mechanical and electrical characteristics at high temperatures and useful as materials for lamination and molding.

In these compositions it is preferred that the number of active hydrogen atoms H in the terminal functional group X of imide compound (B) is from 0.6 to 1.0 per 1 double bond in polymaleimide compound (C).

This resin composition can also be used together with fillers, flame retardants, reinforcing agents, surface-treating agents or pigments as need arises.

The present invention will be illustrated in more detail with reference to the following examples.

Synthetic example 1 Synthesis of material $B_1$

To a 500-ml four-necked flask equipped with a stirrer, thermometer and condenser were charged 110.3 g

(1.125 moles) of maleic anhydride, 3.90 g of N,N'-diphenyl-1,4-phenylenediamine, 100 g of toluene and 50 g of methyl isobutyl ketone. Subsequently, the temperature was raised to 120°C, and 59.1 g (0.5 mole) of α-methylstyrene was added dropwise over 8 hours while maintaining this temperature, after which this temperature was maintained for 2 more hours. After reaction, on cooling the reaction solution with addition of 50 g of toluene and 25 g of methyl isobutyl ketone, crystals precipitated. The crystals were filtered off, washed with toluene several times and dried to obtain 55.7 g of nearly white powdery crystals.

The purity of these $B_1$ crystals by means of GPC was 97.2%, the isomer ratio Y/Z by means of GC was 0.45/0.55, and the melting point was 206-208°C.

Synthetic example 2

To a 500-ml four-necked flask equipped with a stirrer, thermometer and condenser were charged 98.1 g (1 mole) of maleic anhydride, 3.90 g of N,N'-diphenyl-1,4-phenylenediamine and 150 g of xylene. Subsequently, the temperature was raised to 145°C, and 59.1 g (0.5 mole) of α-methylstyrene was added dropwise over 8 hours at the same temperature, after which the temperature was maintained for 2 more hours. After reaction, on cooling the reaction solution with addition of 75 g of methyl isobutyl ketone, crystals precipitated. The crystals were filtered off, washed with toluene several times and dried to obtain 50.4 g of nearly white powdery crystals of $B_1$.

The purity of these crystals by means of GPC was 96.1%, the isomer ratio Y/Z by means of GC was 0.17/0.83, and the melting point was 208-210°C.

Synthetic example 3

To a 500-ml four-necked flask equipped with a stirrer, thermometer and condenser were charged 98.1 g (1 mole) of maleic anhydride, 4.51 g of N-phenyl-N'-isopropyl-1,4-phenylenediamine, 120 g of toluene and 30 g of methyl isobutyl ketone. Subsequently, the temperature was raised to 120°C, and 59.1 g (0.5 mole) of α-methylstyrene was added dropwise over 2 hours at the same temperature, after which the same temperature was maintained for another 8 hours. After reaction, on cooling the reaction solution with addition of 60 g of toluene and 15 g of methyl isobutyl ketone, crystals precipitated. The crystals were filtered off, washed with toluene several times and dried to obtain 67.2 g of nearly white powdery crystals of $B_1$.

The purity of these crystals by means of GPC was 97.8%, the isomer ratio Y/Z by means of GC was 0.66/0.34, and the melting point was 181-183°C.

Example 1

To a flask equipped with a stirrer, thermometer and separator were charged 26.2 g (0.215 mole) of 2,4-tolylenediamine and 117 g of m-cresol, and after raising the temperature to 70°C to dissolve 2,4-tolylenediamine, 45.0 g (0.143 mole) of the material obtained in Synthetic example 1 was added to form a polyamide acid. Thereafter, 25.2 g of toluene was added, and after raising the temperature to 150°C, dehydration was continued for 10 hours at the same temperature.

After reaction, the resulting resin solution was added to 750 g of isopropanol to form precipitates which were then washed twice and dried under reduced pressure to obtain an imide compound. The amine equivalent of this compound was 498 g/eq, and the melting point thereof was about 260°C.

Example 2

To a flask equipped with a stirrer, thermometer and separator were charged 44.8 g (0.143 mole) of the material obtained in Synthetic example 1, 161 g of m-cresol and 8.68 g (0.0714 mole) of 2,4-tolylenediamine, and reaction was carried out at a temperature of 70°C for 1 hour. Subsequently, 15.5 g (0.143 mole) of m-aminophenol was added, and reaction was carried out at the same temperature for 1 hour. Thereafter, 32.2 g of xylene was added, and dehydration was continued at a temperature of 170°C for 6 hours.

After reaction, the resulting resin solution was added to 550 g of isopropanol to form precipitates which were then washed twice and dried under reduced pressure to obtain an imide compound. The hydroxyl equivalent of this compound was 473 g/eq, and the melting point thereof was 270°C.

Example 3

An imide compound was obtained under the same conditions as in Example 1 except that 26.2 g (0.215

mole) of 2,4-tolylenediamine was replaced by 19.3 g (0.0971 mole) of 4,4'-diaminodiphenylmethane, and 45.0 g (0.143 mole) of the material obtained in Syntheticexample 1 was replaced by 26.7 g (0.085 mole) of the material obtained in Synthetic example 2. The amine equivalent of this compound was 1690 g/eq, and the melting point thereof was not less than 300°C.

Example 4

An imide compound was obtained under the same conditions as in Example 2 except that 44.8 g (0.143 mole) of the material obtained in Synthetic example 1 was replaced by 32.0 g (0.102 mole) of the material obtained in Synthetic example 2, 8.68 g (0.0714 mole) of 2,4-tolylenediamine was replaced by 12.9 g (0.0639 mole) of 4,4'-diaminodiphenylmethane, and that the amount of m-aminophenol was changed to 8.30 g (0.0761 mole). The hydroxyl equivalent of this compound was 702 g/eq, and the melting point thereof was about 270°C.

Example 5

An imide compound was obtained under the same conditions as in Example 1 except that the material obtained in Synthetic example 1 was replaced by the material obtained in Synthetic example 3. The amine equivalent of this compound was 506 g/eq, and the melting point thereof was about 260°C.

Example 6

An imide compound was obtained under the same conditions as in Example 2 except that the material obtained in Synthetic example 1 was replaced by the material obtained in Synthetic example 3. The hydroxyl equivalent of this compound was 478 g/eq, and the melting point thereof was about 260°C.

The imide compounds obtained in Examples 1 to 6 are soluble in solvents such as acetone, MEK, methylene chloride or methyl Cellosolve®, and also their compatibility with epoxy resins is good.

Example 7

An imide compound was obtained under the same condition as in Example 1 except that 26.2 g (0.215 mole) of 2,4-tolylenediamine was replaced by 11.9 g (0.0971 mole) of 2,4'-tolylenediamine and 45.0 g (0.143 mole) of the material obtained in Synthetic example 1 was replaced by 26.7 g (0.085 mole) of the material obtained in Synthetic example 3. The amine equivalent of this compound was 1480 g/eq, and the melting point thereof was not less than 300°C.

Examples 8 and 9

Sumi® epoxy ELA-128 (bisphenol A type epoxy resin having an epoxy equivalent of 187 g/eq ; product of Sumitomo Chemical Co., Ltd.) was blended separately with the imide compounds of Examples 1 and 5 in proportions shown in Table 1. The resulting blends were each uniformly dissoved in dimethylformamide and impregnated into glass cloth (WE18K, BZ-2 ; products of Nitto boseki K.K.) which was then treated for 5 minutes in a 180°C oven to obtain prepregs. Six pieces of each prepreg and copper foil (TAI-treated foil 35 μm thick ; product of Furukawa Circuit Foil Co., Ltd.) were piled up and press-molded, at a temperature of 180°C for 5 hours under a pressure of 50 $kg/cm^2$, into respective copper-clad laminates 1 mm thick. The physical properties of these laminates were measured according to JIS-C-6481 to obtain the results shown in Table 1.

Comparative example 1

240 Grams of Sumi® epoxy ESA-011 (bisphenol A type epoxy resin having an epoxy equivalent of 489 g/eq ; product of Sumitomo Chemical Co., Ltd.), 20 g of Sumi® epoxy ESCN-220 (o-cresol novolak type epoxy resin having an epoxy equivalent of 210 g/eq ; product of Sumitomo Chemical Co., Ltd.), 9 g of dicyandiamide and 1 g of 2-phenyl-4-methyl-5-hydroxymethylimidazole were dissolved in a mixed solvent comprising 40 g of dimethylformamide, 60 g of ethylene glycol monomethyl ether and 60 g of methyl ethyl ketone. In the same manner as in Example 8, this solution was impregnated into glass cloth, and the glass cloth was treated for 5 minutes in a 160°C oven to obtain a prepreg which was then press-molded into a laminate. The physical properties of this laminate are shown in Table 1.

Table 1

| Example | | Example 8 | Example 9 | Comparative example 1 |
|---|---|---|---|---|
| Sumi®epoxy ELA-128 (g) | | 100 | 100 | |
| Imide compound in Example 1 (g) | | 133 | – | |
| Imide compound in Example 5 (g) | | – | 135 | |
| $T_g$ | °C | 198 | 201 | 124 |
| Expansion coefficient of Z axis (not higher than $T_g$) | 1/°C | $3.5x10^{-5}$ | $3.5x10^{-5}$ | $5.7x10^{-5}$ |
| Expansion coefficient of Z axis (20°– 200°C) | % | 1.06 | 1.09 | 3.15 |
| Expansion coefficient of Z axis (20°– 260°C) | % | 2.38 | 2.22 | 4.63 |
| Water absorption (after 24 hours' boiling) | % | 1.34 | 1.29 | 1.94 |
| Water absorption (after 48 hours' boiling) | % | 1.47 | 1.44 | 2.18 |
| Peeling strength of copper foil | kg/m | 228 | 239 | 210 |
| Solder resistance (300°C) | Appearance | Pass | Pass | Blister |
| Gelation time (170°C) | % | 16 | 15 | – |

Examples 10 and 11

100 Grams of Sumi® epoxy ESCN-195XL (o-cresol novolak type epoxy resin having an epoxy equivalent of 197 g/eq ; product of Sumitomo Chemical Co., Ltd.), 25 g of the imide compound of Example 2 (Example 10) or 4 (Example 11), 51 g of a phenol novolak resin, 1 g of 2,4,6-tris(dimethylaminomethyl)phenol, 1 g of carnauba wax, 2 g of a silane coupling agent (Toray Silicone SH-6040) and 420 g of silica were kneaded on a two-roll mill for 5 minutes, cooled and pulverized to obtain molding materials. Each molding material was press-molded at a temperature of 170°C for 5 minutes under a pressure of 70 kg/cm$^2$. On placing the press-molded product in a 180°C oven, it hardened after 5 hours. The physical properties of the cured products are shown in Table 2.

Comparative example 2

A hardened product was obtained in the same manner as in Example 10 except that 56 g of the phenol novolak resin alone was used as a hardener and 364 g of silica was used as a filler.

The physical properties of the cured product are shown in Table 2.

Table 2

| Example | | Example 10 | Example 11 | Comparative example 2 |
|---|---|---|---|---|
| $T_g$ | °C | 192 | 194 | 170 |
| Expansion coefficient ($< T_g$) | 1/°C | $2.0 \times 10^{-5}$ | $2.0 \times 10^{-5}$ | $2.6 \times 10^{-5}$ |
| Expansion coefficient (20°- 260°C) | % | 1.12 | 1.20 | 1.35 |
| Flexural strength ( 20°C) | $kg/mm^2$ | 14.9 | 14.7 | 14.8 |
| Flexural strength (100°C) | " | 14.0 | 13.6 | 12.1 |
| Flexural strength (150°C) | " | 12.9 | 12.1 | 9.0 |
| Flexural strength (180°C) | " | 8.8 | 7.4 | 2.1 |

Examples 12, 13 and 14

Sumi® epoxy ELA-128 (bisphenol A type epoxy resin having an epoxy equivalent of 186 g/eq ; product of Sumitomo Chemical Co., Ltd.), the imide compound obtained in Example 1 and N,N'-diphenylmethane bismaleimide (hereinafter referred to as BMI ; product of Sumitomo Chemical Co., Ltd.) were blended in proportions shown in Table 3. The resulting blend was uniformly dissolved in dimethylformamide and impregnated into glass cloth (WE18K, BZ-2 ; products of Nitto Boseki K.K.) which was then treated for 5 minutes in a 180°C oven to obtain a prepreg. Six pieces of this prepreg and copper foil (TAI-treated foil 35 μm thick ; product of Furukawa Circuit Foil Co., Ltd.) were piled up and press-molded, at a temperature of 200°C for 5 hours under a pressure of 50 $kg/cm^2$, into a copper-clad laminate 1 mm thick. The physical properties of this laminate were measured according to JIS-C-6481 to obtain the results shown in Table 3.

Table 3

| Example | | | Example 12 | Example 13 | Example 14 | Comparative example 1 |
|---|---|---|---|---|---|---|
| Sumi® epoxy ELA-128 | | (g) | 100 | 100 | 100 | – |
| Imide compound in Example 1 | | (g) | 166 | 205 | 275 | – |
| BMI | | (g) | 33 | 66 | 125 | – |
| $T_g$ | | °C | 267 | 276 | 273 | 124 |
| Peeling strength of copper foil | | kg/m | 205 | 195 | 171 | 210 |
| Water absorption (after 24 hours' boiling) | | % | 1.26 | 1.35 | 1.37 | 1.94 |
| Water absorption (after 48 hours' boiling) | | % | 1.34 | 1.42 | 1.43 | 2.18 |
| Solder resistance (300°C) | Normal state | Appearance | Pass | Pass | Pass | Blister |
| | After 2 hours' boiling | Appearance | Pass | Pass | Pass | Blister |

Examples 15 to 19

Sumi® epoxy ELA-128 (bisphenol A type epoxy resin having an epoxy equivalent of 186 g/eq ; product of Sumitomo Chemical Co., Ltd.), Sumi® epoxy ESB-400 (brominated bisphenol A type epoxy resin having an epoxy equivalent of 400 g/eq ; product of Sumitomo Chemical Co., Ltd.), the imide compound obtained in Example 1 and o-cresol novolak resin (softening point, 105°C) or bisphenol A were blended in proportions shown in Table 4. The resulting blends were each uniformly dissolved in dimethylformamide and impregnated into glass cloth (WE18K, BZ-2 ; products of Nitto Boseki K.K.) which was then treated for 5 minutes in a 180°C oven to obtain prepregs. Six pieces of each prepreg and copper foil (TAI-treated foil 35 µm thick ; product of Furukawa Circuit Foil Co., Ltd.) were piled up and press-molded, at a temperature of 180°C for 5 hours under a pressure of 50 $kg/cm^2$, into copper-clad laminates 1 mm thick. The physical properties of these laminates were measured according to JIS-C-6481 to obtain the results shown in Table 4.

Table 4

| Example | | | Example 15 | Example 16 | Example 17 | Example 18 | Example 19 | Comparative example 1 |
|---|---|---|---|---|---|---|---|---|
| Sumi® epoxy ELA-128 | | (g) | 55 | 55 | 55 | 55 | 55 | |
| Sumi® epoxy ESB-400 | | (g) | 45 | 45 | 45 | 45 | 45 | |
| Imide compound in Example 1 | | (g) | 64.2 | 72.2 | 64.2 | 64.1 | 72.2 | |
| o-Cresol novolak resin | | (g) | 15.3 | 11.5 | 10.2 | 7.7 | — | |
| Bisphenol A | | (g) | — | — | — | — | 9.4 | |
| Peeling strength of copper foil | | kg/m | 195 | 205 | 212 | 215 | 203 | 210 |
| Water absorption (after 24 hours' boiling) | | % | 1.15 | 1.20 | 1.10 | 1.20 | 1.17 | 1.94 |
| Water absorption (after 48 hours' boiling) | | % | 1.27 | 1.27 | 1.17 | 1.29 | 1.25 | 2.18 |
| Solder resistance (300°C) | Normal state | Appearance | Pass | Pass | Pass | Pass | Pass | Blister |
| | After 2 hours' boiling | Appearance | Pass | Pass | Pass | Pass | Pass | Blister |
| $T_g$ | | °C | 210 | 215 | 210 | 220 | 215 | 124 |
| Gelation time (170°C) | | min. | 6 | 7.5 | 8 | 10 | 8 | — |

Examples 20 and 21

Sumi® epoxy ELA-128 (bisphenol A type epoxy resin having an epoxy equivalent of 186 g/eq ; product of Sumitomo Chemical Co., Ltd.), Sumi® epoxy ESB-400 (brominated bisphenol A type epoxy resin having an epoxy equivalent of 400 g/eq ; product of Sumitomo Chemical Co., Ltd.), the imide compound obtained in Example 1, o-cresol novolak resin (softening point, 105°C) and N,N'-diphenylmethane bismaleimide (hereinafter referred to as BMI ; product of Sumitomo Chemical Co., Ltd.) were blended in proportion shown in Table 5. The resulting blends were each uniformly dissolved in dimethylformamide and impregnated into glass cloth (WE18K, BZ-2 ; products of Nitto Boseki K.K.) which was then treated for 5 minutes in a 180°C oven to obtain prepregs. Six pieces of each prepreg and copper foil (TAI-treated foil 35 μm thick ; product of Furukawa Circuit Foil Co. Ltd.) were piled up and press-molded, at a temperature of 180°C for 5 hours under a pressure of 50 kg/cm$^2$, into copper-clad laminates 1 mm thick. The physical properties of these laminates were measured according to JIS-C-6481 to obtain the results shown in Table 5.

TAble 5

| Example | | | Example 20 | Example 21 | Comparative example 1 |
|---|---|---|---|---|---|
| Sumi® epoxy ELA-128 | | (g) | 55 | 55 | |
| Sumi® epoxy ESB-400 | | (g) | 45 | 45 | |
| Imide compound in Example 1 | | (g) | 120 | 108 | |
| o-Cresol novolak resin | | (g) | 9 | 9 | |
| BMI | | (g) | 30 | 20 | |
| Peeling strength of copper foil | | kg/m | 190 | 200 | 210 |
| Water absorption (after 24 hours' boiling) | | % | 1.31 | 1.20 | 1.94 |
| Water absorption (after 48 hours' boiling) | | % | 1.39 | 1.27 | 2.18 |
| Solder resistance (300°C) | Normal state | Appearance | Pass | Pass | Blister |
| | After 2 hours' boiling | Appearance | Pass | Pass | Blister |
| $T_g$ | | °C | 250 | 256 | 124 |
| Gelation time (170°C) | | min | 3.5 | 4.5 | – |

Examples 22 and 23

Sumi® epoxy ELA-128 (bisphenol A type epoxy resin having an epoxy equivalent of 186 g/eq ; product of Sumitomo Chemical Co., Ltd.) and the imide compound of Example 1 (Example 22) or 5 (Example 23) were blended in proportions shown in Table 6 and kneaded on a mixing roll at a temperature of from 110° to 50°C for 10 minutes to obtain adhesive compositions. The adhesive compositions were each coated in a molten state onto two pieces of soft steel plate, 1.6 mm thick, 25 mm wide and 100 mm long, previously surface-polished. rinsed and defatted according to JIS-K-6850. Two pieces of the soft steel plate were then adhered to each other so that the adhesion area was 15 mm × 25 mm and hardened by applying heat-treatment at a temperature of 200°C for 2 hours under a pressure of about 3 kg/cm$^2$. Thus, five test pieces were prepared for each adhesive composition. Using these test pieces, overlap shear strength at temperatures of 20°C, 100°C, 150°C and 200°C was measured. The results are shown in Table 6.

Comparative example 3

100 Grams of Sumi® epoxy ESA-011 (bisphenol A type epoxy resin having an epoxy equivalent of 478 g/eq ; product of Sumitomo Chemical Co., Ltd.), 4 g of dicyandiamide, 1.5 g of 2-ethyl-4-methylimidazole and 17 g of Hycar® CTBN 1300 × 13 (B, F ; products of Goodrich Chemical Co., Ltd.), which was a nitrile rubber component, were blended. The blend was measured for overlap shear strength in the same manner as in Example 22 except that the heat-treatment was carried out at 140°C for 3 hours. The result is shown in Table 6.

Comparative example 4

100 Grams of Sumi® epoxy ELM-434 (glycidylamine type epoxy resin having an epoxy equivalent of 120 g/eq ; product of Sumitomo Chemical Co., Ltd.), which was a polyfunctional epoxy resin, 47.9 g of 4,4′-diaminodiphenylsulfone and 1 g of boron trifluoride/monoethylamine complex were blended. The blend was measured for overlap shear strength in the same manner as in Example 22. The result is shown in Table 6.

Comparative example 5

Using Kenimid® 601S (product of Nippon Polyimide K.K.), overlap shear strength was measured in the same manner as in Example 22 except that the heat-treatment was carried out at 200°C for 5 hours. The result is shown in Table 6.

Table 6

| Example | | Example 22 | Example 23 | Comparative example 3 | Comparative example 4 | Comparative example 5 |
|---|---|---|---|---|---|---|
| Sumi® epoxy ELA-128 (g) | | 100 | 100 | | | |
| Imide compound in Example 1 (g) | | 133 | – | | | |
| Imide compound in Example 5 (g) | | – | 135 | | | |
| Overlap shear strength ($kg/cm^2$) | 20°C | 200 | 204 | 280 | 141 | 69 |
| | 100°C | 164 | 169 | 140 | 141 | 70 |
| | 150°C | 155 | 157 | 30 | 140 | 61 |
| | 200°C | 144 | 148 | 17 | 132 | 60 |

Examples 24 and 25

Sumi® epoxy ELA-128 (bisphenol A type epoxy resin having an epoxy equivalent of 186 g/eq ; product of Sumitomo Chemical Co., Ltd.) was blended separately with the imide compounds of Examples 2 and 4 in proportions shown in Table 7 and kneaded on a mixing roll at a temperature of from 110° to 50°C for 10 minutes to obtain adhesive compositions. Sixty grams of each composition was dissolved in 40 g of dimethylformamide and impregnated into glass cloths (WE116E, BY52 ; product of Nitto Boseki K.K.) which were then treated for 5 minutes in a 180°C oven to prepare prepregs.

Each prepreg was cut into a size of 25 mm (width) × 180 mm (length), put between two pieces of aluminum alloy (JIS-A-2017), 25 mm (width) × 200 mm (length) × 0.5 mm (thickness), previously surface-polished, rinsed and defatted, and adhered and hardened by applying heat-treatment at a temperature of 200°C for 2 hours under a pressure of 15 kg/cm$^2$. Thus, five test pieces were prepared for each adhesive composition. Using these test pieces, peeling strength was measured at temperatures of 20°C, 100°C, 150°C and 200°C according to JIS-K-6854. The results are shown in Table 7.

Comparative example 6

In the same manner as in Example 24, a varnish, prepared by dissolving 60 g of the adhesive composition obtained in Comparative example 3 in 40 g of methyl Cellosolve®, was impregnated into glass cloth which was then treated at a temperature of 150°C for 5 minutes to prepare a prepreg. Thereafter, peeling strength was measured in the same manner as in Example 24 except that the heat-treatment was carried out at of 140°C for 3 hours. The result is shown in Table 7.

Comparative example 7

Sixty grams of the adhesive composition obtained in Comparative example 4 was dissolved in 40 g of dimethylformamide, and treatment after that was carried out in the same manner as in Example 24 to measure peeling strength. The result is shown in Table 7.

Comparative example 8

Sixty grams of Kerimid® 601S was dissolved in 40 g of dimethylformamide, and treatment after that was carried out in the same manner as in Example 24 except that the heat-treatment was carried out at a temperature of 200°C for 5 hours, to measure peeling strength. The result is shown in Table 7.

It is apparent from Tables 6 and 7 that the adhesive compositions of the present invention have excellent thermal resistance and adhesion.

Table 7

| Example | | Example 24 | Example 25 | Comparative example 6 | Comparative example 7 | Comparative example 8 |
|---|---|---|---|---|---|---|
| Sumi® epoxy ELA-128 (g) | | 100 | 100 | | | |
| Imide compound in Example 2 (g) | | 228 | — | | | |
| Imide compound in Example 4 (g) | | — | 337 | | | |
| peeling strength (kg/cm) | 20°C | 3.4 | 3.5 | 5.1 | 0.32 | 0.53 |
| | 100°C | 3.4 | 3.5 | 4.6 | 0.23 | 0.36 |
| | 150°C | 3.5 | 3.5 | 1.4 | 0.19 | 0.25 |
| | 200°C | 3.1 | 3.2 | 0.6 | 0.16 | 0.27 |

Examples 26, 27 and 28

N,N'-diphenylmethane bismaleimide (BMI ; product of Sumitomo Chemical Co., Ltd.), the imide compound obtained in Example 1 and Sumi® epoxy ELA-128 (bisphenol A type epoxy resin having an epoxy equivalent of 186 g/eq ; product of Sumitomo Chemical Co., Ltd.) were blended in proportions shown in Table 8. Each resulting blend was mixed with heating and press-molded at of 200°C for 5 hours. The physical properties of the cured products obtained are shown in Table 8.

Comparative example 9

A resin blend comprising 1 mole of BMI and 0.4 mole of diaminodiphenylmethane (hereinafter referred to as DDM) was press-molded at 230°C for 5 hours in the same manner as in Example 26, to obtain a cured product. The physical properties of this product are shown in Table 8.

Table 8

| Example | | | Example 26 | Example 27 | Example 28 | Comparative example 9 |
|---|---|---|---|---|---|---|
| BMI | | (g) | 100 | 100 | 100 | 100 |
| Imide compound in Example 1 | | (g) | 117.1 | 87.8 | 122.2 | |
| Sumi® epoxy ELA-128 | | (g) | | | 11.5 | |
| DDM | | (g) | | | | 22.1 |
| Physical properties of cured product | $T_g$ | °C | 267 | 266 | 255 | 250 |
| | Expansion coefficient $\alpha_1$* | $°C^{-1}$ | $4.8x10^{-5}$ | $5.15x10^{-5}$ | $5.2x10^{-5}$ | $4.8x10^{-5}$ |
| | Expansion * (260°C/25°C) | % | 1.2 | 1.39 | 1.6 | 1.9 |
| | Flexural modulus | $kg/mm^2$ | 452 | 448 | 420 | 410 |
| Gelation time (180°C) | | min | 12 | 12 | 10 | 20 |

* Measured by the TMA method (by means of DT-40 thermal analyzer; product of Shimadzu Seisakusho Co., Ltd.)

Other measurement values were obtained according to JIS K 6911.

## Claims

1. An imide compound of formula (I),

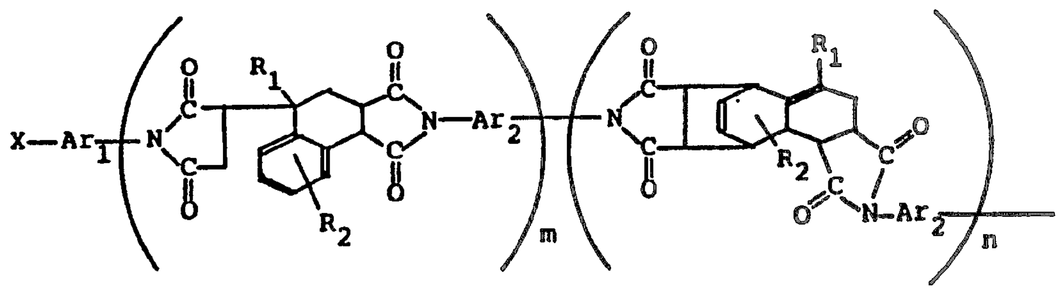

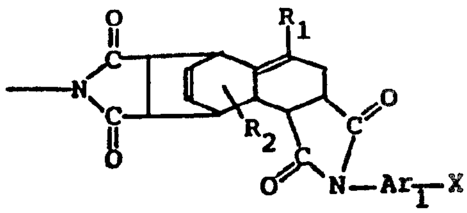

(I)

wherein each X is selected from $-NH_2$ and $-OH$ groups ; each $Ar_1$ and each $Ar_2$ is selected independently from aromatic residues ; each $R_1$ is selected from a hydrogen atom and $C_1$-$C_{10}$ alkyl groups, each $R_2$ is selected from a hydrogen atom and hydroxyl and $C_1$-$C_{20}$ alkyl and $C_1$-$C_{20}$ alkoxy groups ; m is 0 or a number up to 30, and n is 0 or a number up to 30.

2. An imide compound according to Claim 1, wherein each of m and n represents 0 or a number up to 8.

3. An imide compound according to claim 1 or 2 wherein each $R_1$ is selected from alkyl groups having from 1 to 3 carbon atoms.

4. An epoxy resin composition comprising epoxy resin(s) (A) and imide compound(s) (B) according to any of claims 1 to 3.

5. A composition according to claim 4 also containing polymaleimide compound(s) (C) having two or more maleimide groups in the molecule and/or compound(s) (D) having two or more phenolic –OH groups in the molecule.

6. A composition according to claim 5 containing polymaleimide compound (C) selected from N,N'-diphenylmethane bismaleimide and N,N'-diphenylether bismaleimide.

7. A composition according to claim 5 or 6 wherein the proportions of (A), (B) and (C) are such that there is from 0.6 to 1.2 gram equivalent of (B) per 1 gram equivalent of (A), the number of active hydrogen atoms H in the terminal functional group X of (B) is from 0.6 to 1 per 1 double bond in (C), and the weight ratio (A)/(C) is from 95/5 to 40/60.

8. A composition according to claim 5 wherein the ratio of the number of active hydrogen atoms in the terminal functional groups X of imide compound (B) to the number of –OH groups in the polyphenol compound (D), i.e. (B)/(D), is from 2/1 to 4/1, and the sum of active hydrogen equivalents in (B) and (D) is from 0.6 to 1.2 gram equivalent per 1 gram equivalent of epoxy resin (A).

9. An adhesive composition which comprises as essential components epoxy resin(s) (A) and imide compound(s) (B) according to any of claims 1 to 3.

10. A thermosetting resin composition which comprises polymaleimide compound(s) (C) having two or more maleimide groups in the molecule and imide compound(s) (B) according to any of claims 1 to 3.

## Revendications

1. Imide de formule (I)

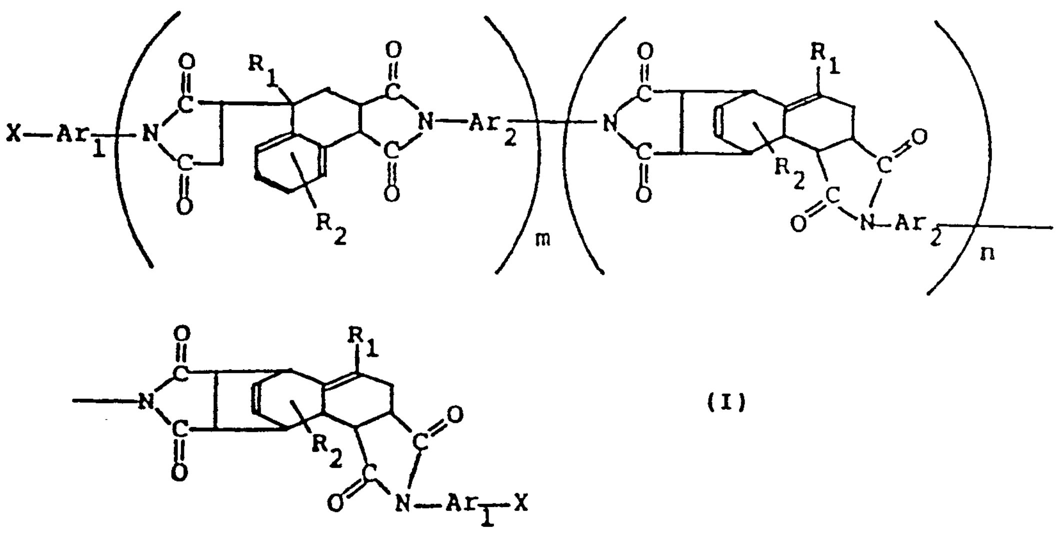

(I)

dans laquelle chaque groupe X est choisi entre des groupes $-NH_2$ et $-OH$ ; chaque groupe $Ar_1$ et chaque groupe $Ar_2$ sont choisis, indépendamment, entre des résidus aromatiques ; chaque groupe $R_1$ est choisi entre un atome d'hydrogène et des groupes alkyle en $C_1$ à $C_{10}$, chaque groupe $R_2$ est choisi entre un atome d'hydrogène, un groupe hydroxyle et des groupes alkyle en $C_1$ à $C_{20}$ et alkoxy en $C_1$ à $C_{20}$ ; m est égal à 0 ou à un nombre allant jusqu'à 30, et n est égal à 0 ou à un nombre allant jusqu'à 30.

2. Imide suivant la revendication 1, dans lequel chacun des indices m et n est égal à 0 ou représente un nombre allant jusqu'à 8.

3. Imide suivant la revendication 1 ou 2, dans lequel chaque groupe $R_1$ est choisi entre des groupes alkyle ayant 1 à 3 atomes de carbone.

4. Composition de résine époxy comprenant une ou plusieurs résines époxy (A) et un ou plusieurs imides (B) suivant l'une quelconque des revendications 1 à 3.

5. Composition suivant la revendication 4, contenant également un ou plusieurs polymaléimides (C) ayant deux ou plus de deux groupes maléimide dans la molécule et/ou un ou plusieurs composés (D) ayant deux ou plus de deux groupes –OH phénoliques dans la molécule.

6. Composition suivant la revendication 5, contenant un polymaléimide (C) choisi entre le N,N'-diphénylméthane-bis-maléimide et le N,N'-diphényléther-bis-maléimide.

7. Composition suivant la revendication 5 ou 6, dans laquelle les proportions de (A), (B) et (C) sont choisies de manière qu'une quantité de 0,6 à 1,2 équivalent-gramme de composé (B) soit présente par équivalent-gramme de composé (A), que le nombre d'atomes d'hydrogène actif (H) dans le groupe fonctionnel terminal X du composé (B) soit de 0,6 à 1 par double liaison dans le composé (C) et que le rapport pondéral (A)/(C) soit compris dans l'intervalle de 95/5 à 40/60.

8. Composition suivant la revendication 5, dans laquelle le rapport du nombre d'atomes d'hydrogène actif dans les groupes fonctionnels terminaux X de l'imide (B) au nombre de groupes –OH dans le polyphénol (D), à savoir (B)/(D), est compris dans l'intervalle de 2/1 à 4/1, et la somme des équivalents d'hydrogène actif dans les composés (B) et (D) est comprise dans l'intervalle de 0,6 à 1,2 équivalent-gramme par équivalent-gramme de résine époxy (A).

9. Composition adhésive qui comprend, comme constituants essentiels, une ou plusieurs résines époxy (A) et un ou plusieurs imides (B) suivant l'une quelconque des revendications 1 à 3.

10. Composition de résine thermodurcissable qui comprend un ou plusieurs polymaléimides (C) ayant deux ou plus de deux groupes maléimide dans la molécule et un ou plusieurs imides (B) suivant l'une quelconque des revendications 1 à 3.

**Ansprüche**

1. Imid-Verbindung der Formel (I)

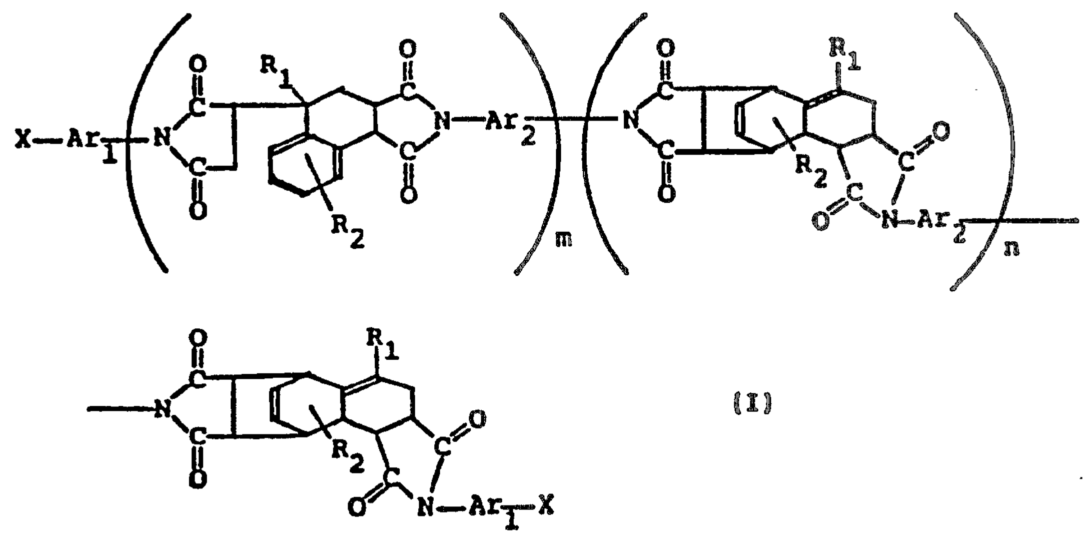

(I)

in der X jeweils eine $-NH_2$ oder eine $-OH$-Gruppe ist, $Ar_1$ und $Ar_2$ jeweils unabhängig voneinander ein aromatischer Rest ist, $R_1$ jeweils ein Wasserstoffatom oder ein $C_1$-$C_{10}$ Alkylrest ist, $R_2$ jeweils ein Wasserstoffatom, eine Hydroxylgruppe, ein $C_1$-$C_{20}$ Alkylrest oder ein $C_1$-$C_{20}$ Alkoxyrest ist, m 0 oder eine Zahl bis 30 ist und n 0 oder eine Zahl bis 30 ist.

2. Imid-Verbindung nach Anspruch 1, bei der jeweils m und n 0 oder eine Zahl bis 8 ist.

3. Imid-Verbindung nach Anspruch 1 oder 2, bei der $R_1$ jeweils ein Alkylrest mit 1 bis 3 C-Atomen ist.

4. Epoxyharz-Zusammensetzung, enthaltend Epoxyharz(e) (A) und Imidverbindung(en) (B) nach einem der Ansprüche 1 bis 3.

5. Zusammensetzung nach Anspruch 4, enthaltend ferner Polymaleinimid-Verbindung(en) (C) mit zwei oder mehr Maleinimid-Gruppen im Molekül und/oder Verbindung(en) (D) mit zwei oder mehr Phenol $-OH$ Gruppen im Molekül.

6. Zusammensetzung nach Anspruch 5, enthaltend eine aus N,N'-Diphenylmethanbismaleinimid und N,N'-Diphenyletherbismaleinimid ausgewählte Polymaleinimid-Verbindung (C).

7. Zusammensetzung nach Anspruch 5 oder 6, bei der die Mengenverhältnisse von (A), (B) und (C) derart sind, daß pro Grammäquivalent von (A) 0,6 bis 1,2 Grammäquivalente von (B) vorliegen, die Zahl der aktiven Wasserstoffatome H im endständigen funktionellen Rest X von (B) von 0,6 bis 1 pro 1 Doppelbindung in (C) beträgt und das Gewichtsverhältnis von (A)/(C) von 95/5 bis 40/60 beträgt.

8. Zusammensetzung nach Anspruch 5, bei der das Verhältnis der Zahl der aktiven Wasserstoffatome in den endständigen funktionellen Resten X der Imid-Verbindung (B) zu der Zahl der $-OH$ Gruppen in der Polyphenol-Verbindung (D), nämlich (B)/(D), von 2/1 bis 4/1 beträgt und die Summe aktiver Wasserstoff-Äquivalente in (B) und (D) von 0,6 bis 1,2 Grammäquivalent pro Grammäquivalent Epoxyharz (A) beträgt.

9. Kleber-Zusammensetzung, umfassend als wesentliche Komponenten Epoxyharz(e) (A) und Imid-Verbindung(en) (B) nach einem der Ansprüche 1 bis 3.

10. Hitzehärtbare Harz-Zusammensetzung, umfassend Polymaleinimid-Verbindung(en) (C) mit zwei oder mehr Maleinimid-Gruppen im Molekül und Imid-Verbindung(en) (B) nach einem der Ansprüche 1 bis 3.